# EUROPEAN PATENT APPLICATION

(11) **EP 4 725 591 A1**
(43) Date of publication of application: **15.04.2026**
(21) Application number: 24206308.9
(22) Date of filing: 14.10.2024
(51) Int. Cl.: B01F 35/22, G01N 21/3563, A01K 5/00, A23N 17/00, G01N 21/3554, G01N 21/85, G01N 21/359, G01N 21/31, B01F 101/18

(54) **DETERMINING THE DEGREE OF MIXING OF A FODDER MIXTURE WITH A SPECTRAL SENSOR**

(71) Applicant: Trioliet B.V., 7575 BW Oldenzaal (NL)
(72) Inventor: Liet, Robert Jan, 7573 CS Oldenzaal (NL)
(74) Representative: Grünecker Patent- und Rechtsanwälte PartG mbB

(57) **Abstract**

The invention provides a method for determining a current homogeneity parameter of a fodder mixture for farm animals while mixing the fodder mixture in a mixing container of a fodder mixing apparatus, wherein the fodder mixture comprises at least two different fodder components, comprising:
- recording, in sequence, optical spectra of the fodder mixture with a spectral sensor while mixing, wherein the method further comprises the steps of:
a) receiving, by an electronic control unit, at least three optical spectra recorded by the spectral sensor,
b) calculating, by the electronic control unit, a correlation between the at least three optical spectra, and
c) determining, by the electronic control unit, the current homogeneity parameter based on the correlation between the at least three optical spectra.

## Description

The invention relates to methods for determining a current homogeneity parameter of a fodder mixture for farm animals while mixing the fodder mixture in a mixing container of a fodder mixing apparatus.

Fodder mixing apparatuses are used to mix different types of fodder components into a fodder mixture, which is to be fed to farm animals. On the one hand, it is important not to mix the fodder mixture for too long, as this can have a negative impact on the quality and texture of the fodder mixture and can cost unnecessary energy. On the other hand, it is important not to mix the fodder mixture too short in order to achieve a homogeneous mixture, in particular, to ensure that every farm animal receives the same fodder mixture.

It is known from utility model DE 20 2010 005 053 U1 that a sensor can be used for determining the degree of mixing of a fodder mixture.

However, there is a need to improve the accuracy in determining the degree of mixing of the fodder mixture.

As a consequence, it is an object of the invention to provide a method for determining a current homogeneity parameter, which particularly allows a more accurate determination of the degree of mixing of the fodder mixture.

This object is achieved by a method according to claim 1 and a method according to claim 5. Embodiments are listed in the dependent claims.

Such methods allow for a more accurate determination of a current homogeneity parameter and thus, a more accurate determination of the degree of mixing of the fodder mixture. Consequently, an optimized duration of mixing of the fodder mixture can be achieved when controlling the mixing process based on the determined current homogeneity parameter or based on a parameter derived therefrom.

As used herein, the term "current homogeneity parameter" can be understood as a value indicative for the current degree of mixing of the fodder mixture, i.e. how homogeneous the fodder mixture is mixed at a particular point in time. The value can, for example, range between values "0" and "1", wherein the value "0" indicates that the fodder mixture is not mixed at all and the value "1" indicates that the fodder mixture is fully mixed. For a fully homogeneous fodder mixture, any two or more samples of fodder taken from the fodder mixture will have the same composition of fodder components.

Further, as used herein, the term "dry matter" can be understood as all constituents of a fodder mixture excluding water. For example, the fodder mixture can consist of its dry matter and water content.

The disclosure according to a first aspect provides a method for determining a current homogeneity parameter of a fodder mixture for farm animals while mixing the fodder mixture in a mixing container of a fodder mixing apparatus, wherein the fodder mixture comprises at least two different fodder components, comprising:
- recording, in sequence, optical spectra of the fodder mixture with a spectral sensor while mixing,
the method further comprising the steps:
a) receiving, by an electronic control unit, at least three optical spectra recorded by the spectral sensor,
b) calculating, by the electronic control unit, a correlation between the at least three optical spectra, and
c) determining, by the electronic control unit, the current homogeneity parameter based on the correlation between the at least three optical spectra.

By determining the current homogeneity parameter based on the correlation between at least three optical spectra, the accuracy of the homogeneity determination can be improved.

The electronic control unit can receive more than three optical spectra recorded by the spectral sensor, in particular five or more optical spectra, in particular ten or more optical spectra. Consequently, the correlation between the optical spectra can be calculated based on more than three optical spectra, in particular five or more optical spectra, in particular ten or more optical spectra. The at least three optical spectra may have been recorded in succession, i.e. recorded directly one after the other. The electronic control unit can receive the at least three optical spectra in sequence or as a batch from the spectral sensor, in particular via a data connection between the spectral sensor and the electronic control unit.

Invalid optical spectra can be discarded, in particular by the electronic control unit. The electronic control unit can be configured to filter valid optical spectra from the at least three optical spectra received by the electronic control unit. The electronic control unit can be configured to use only valid optical spectra for calculating the correlation between the at least three optical spectra. The electronic control unit can particularly be configured to proceed with steps b) and c) only based on at least three valid optical spectra.

An optical spectrum can be considered as invalid, for instance, if the spectral sensor records no fodder mixture or if the spectral sensor is overexposed while recording an optical spectrum. Other examples for an invalid optical spectrum include an optical spectrum with one or more negative intensities and/or an optical spectrum in which the fodder mixture was not illuminated with full intensity by the emitted electromagnetic radiation of the spectral sensor, for example if the lighting for emitting the electromagnetic radiation is defective. Invalid spectra can also occur if the sensor does not detect enough fodder mixture in its detection range so that not enough electromagnetic radiation emitted by the spectral sensor is reflected by the fodder mixture into the sensor.

The term "correlation" can be understood as a measure of the degree of relation or interrelation between two or more variables or between two or more sets of variables. The correlation can be expressed by a correlation coefficient. For example, the correlation, in particular, the correlation coefficient, can indicate the degree of relation between the measured intensities of the detected electromagnetic radiation of the at least three optical spectra. The correlation, in particular the correlation coefficient, can range between values "0" and "1", wherein the value "0" can mean that no correlation between the at least three optical spectra is present and the value "1" can mean a maximum correlation is present between the at least three optical spectra. Other values than "0" and "1" are conceivable as well. The value for the current homogeneity parameter can correspond to the value of the correlation, in particular to the correlation coefficient.

The correlation can be a linear correlation, in particular a Pearson correlation. The Pearson correlation can be expressed by a Pearson correlation coefficient. The linear correlation, in particular the Pearson correlation, in particular the Pearson correlation coefficient, can measure the degree of linear relation or interrelation between the at least three optical spectra. For example, the linear correlation can indicate the degree of linear relation between the measured intensities of the detected electromagnetic radiation of the at least three optical spectra. The calculation of the Pearson correlation can be performed pairwise. For example, a coefficient is calculated for every pair of two optical spectra of the at least three optical spectra and a mean value of the calculated coefficients is determined. Thus, for example, for three optical spectra, three coefficients are calculated and a mean value of the three calculated coefficients is determined and set as the Pearson correlation coefficient for the at least three optical spectra. The Pearson correlation coefficient can range between values "-1" and "+1", wherein the value "-1" means a negative linear correlation, the value "0" means no correlation and the value "+1" means a positive linear correlation. An absolute value of the Pearson correlation coefficient can be calculated. The current homogeneity parameter can correspond to the absolute value of the Pearson correlation coefficient. Thus, a value of "0" can mean that the fodder mixture is not mixed and a value of "1" can mean that the fodder mixture is homogenously mixed.

The correlation can also be a correlation of a higher degree, in particular a correlation of second or third order.

The correlation can alternatively or additionally be based on Lin's concordance correlation coefficient. The calculation of Lin's concordance correlation coefficient can be performed pairwise as well. For example, a coefficient is calculated for every pair of two optical spectra of the at least three optical spectra and a mean value of the calculated coefficients is determined. Thus, for example, for three optical spectra, three coefficients are calculated and a mean value of the three calculated coefficients is determined and set as the Lin's concordance correlation coefficient for the at least three optical spectra. Lin's concordance correlation coefficient can range between values "0" and "1". The current homogeneity parameter can correspond to Lin's concordance correlation coefficient, in particular to the mean value of the calculated Lin's concordance correlation coefficients. A value of "0" can mean that the fodder mixture is not mixed and a value of "1" can mean that the fodder mixture is homogenously mixed. Any values between these two limits indicate a certain degree of mixing.

The method may further use a total homogeneity parameter, in particular for smoothing statistical short-scale variations of the current homogeneity parameter.

The method can particularly further comprise setting, by the electronic control unit, a value of a total homogeneity parameter to a predetermined value, in particular zero, when starting mixing and/or when adding further fodder to the mixing container, in particular a further fodder component, and updating, by the electronic control unit, the value of the total homogeneity parameter by calculating a weighted sum of the current homogeneity parameter and the set value of the total homogeneity parameter.

Since the current homogeneity parameter may be subject to measurement errors or statistical fluctuations, the value of the current homogeneity parameter may not always accurately represent the homogeneity of the complete fodder mixture in the mixing container. In other words, the current homogeneity parameter may be a snapshot of the homogeneity of only a part of the fodder mixture in the mixing container, in particular the part of the fodder mixture probed by the spectral sensor when measuring the at least three optical spectra. Thus, the total homogeneity parameter may be introduced and updated based on subsequent determinations of current homogeneity parameters. The total homogeneity parameter, thus, may represent an average of the current homogeneity parameter of different parts of the fodder mixture measured at different points in time. The temporal evolution of the total homogeneity value, thus, may better represent the development of the actual homogeneity of the fodder mixture in the mixing container.

The method can particularly further comprise repeatedly determining, by the electronic control unit, a new current homogeneity parameter by carrying out the above-mentioned steps a) to c) based on different optical spectra, and updating, by the electronic control unit, the value of the total homogeneity parameter after each determination of a new current homogeneity parameter by calculating a weighted sum of the new current homogeneity parameter and the value of the total homogeneity parameter prior to the update. Each "new current homogeneity parameter" may, thus, correspond to the current homogeneity parameter associated with a particular set of at least three optical spectra from the different optical spectra. Each "new current homogeneity parameter" may be used to update the total homogeneity parameter, in particular by determining a weighted average. In this way, the development over time of the current homogeneity parameter can be taken into account. In addition, the impact of sudden changes of the current homogeneity parameter on the method, in particular statistical outliers, can be reduced. The sets of at least three optical spectra from the different optical spectra used to determine the different new current homogeneity parameters can differ from each other in at least one optical spectrum.

The disclosure according to a second aspect provides a method for determining a current homogeneity parameter of a fodder mixture for farm animals while mixing the fodder mixture in a mixing container of a fodder mixing apparatus, wherein the fodder mixture comprises at least two different fodder components, comprising:
- recording, in sequence, optical spectra of the fodder mixture with a spectral sensor while mixing,
- receiving, by an electronic control unit, at least three optical spectra recorded by the spectral sensor,
- determining, by the electronic control unit, a fodder value for each of the at least three optical spectra using a prediction model, wherein the fodder value is in particular selected from a group comprising carbohydrate, fat, fiber, protein, dry matter and water,
- calculating, by the electronic control unit, an initial standard deviation of the determined fodder values, and
- determining, by the electronic control unit, the current homogeneity parameter based on the initial standard deviation of the determined fodder values.

In other words, a prediction model may be applied repeatedly to different optical spectra to determine a set of fodder values for the fodder mixture. Each fodder value in this set may correspond to the fodder value being determined based on a different one of the at least three optical spectra determined in the mixing process. Thus, the set of fodder values may represent a temporal evolution of the predicted fodder value. The initial standard deviation may be indicative of how the fodder value varies in the set and, thus, may be indicative of the homogeneity. For a homogeneous fodder mixture, the fodder values should be basically constant, i.e. the same for each measurement. Consequently, the standard deviation would be minimal. As long as the fodder is not optimally mixed, the standard deviation will still assume higher values.

In particular the same prediction model may be applied to determine each of the fodder values in the before mentioned set of fodder values.

The electronic control unit can receive the at least three optical spectra from the spectral sensor, in particular via a data connection between the spectral sensor and the electronic control unit. The electronic control unit can receive the at least three optical spectra in sequence as the optical spectra are recorded by the spectral sensor or as a batch from the spectral sensor.

The electronic control unit can receive more than three optical spectra, in particular five or more optical spectra, in particular ten or more optical spectra. The at least three optical spectra may have been recorded in succession, i.e. recorded directly one after the other.

As discussed above for the first aspect, invalid optical spectra may be discarded, i.e. not used for determining the current homogeneity parameter.

The spectral sensor in any of the two described aspects can be an infrared sensor, in particular a near infrared (NIR)-sensor. The spectral sensor can emit electromagnetic radiation, in particular near infrared light, onto a target object and then detect the reflected electromagnetic radiation. The spectral sensor can determine optical spectra based on the frequency of the reflected electromagnetic radiation and corresponding intensities of the reflected electromagnetic radiation. The target object can be the part of the fodder mixture from which the spectral sensor detects the electromagnetic radiation.

The spectral sensor can be arranged on the mixing container of the fodder mixing apparatus or the spectral sensor can be arranged on a mixing device, in particular a mixing auger, of the fodder mixing apparatus. In the case of the spectral sensor being arranged on the mixing device, the optical spectra can be recorded when the fodder mixture is in front of the spectral sensor, for example, when the fodder mixture slides over the spectral sensor while mixing. The fodder mixing apparatus can comprise two or more spectral sensors. A first spectral sensor of the two or more spectral sensors can be arranged on the mixing container. A second spectral sensor of the two or more sensor can be arranged on the mixing device, in particular the mixing auger, of the fodder mixing apparatus.

An optical spectrum, as used herein, can describe a functional relation between the frequency of the detected electromagnetic radiation and a corresponding intensity of the detected electromagnetic radiation. The spectral sensor can detect electromagnetic radiation in a specific frequency range. For example, the spectral sensor can detect electromagnetic radiation in the range of 1300 nm to 2525 nm. The spectral sensor can have a specific resolution, for example, a resolution of 5 nm. The spectral sensor can measure a value for an intensity of the detected electromagnetic radiation for each frequency bin corresponding to the specific resolution, for example, the spectral sensor can detect an intensity value for each range of 5 nm in the frequency range of 1300 nm to 2525 nm. The optical spectra can be displayed as a plot with the frequency of the detected electromagnetic radiation on the x-axis and the detected intensity of the electromagnetic radiation on the y-axis.

The optical spectra can be recorded at predetermined time intervals, for example, an optical spectrum can be recorded every ten seconds. The time between the recordings of optical spectra can be adjusted so that changes in the optical spectra can be detected by the spectral sensor. For example, the time interval can be increased, if a probability of the spectral sensor recording the same spot of the fodder mixture is greater than a predetermined threshold. The time between the recordings of optical spectra can also be adjusted based on a mixing speed of the fodder mixture and/or based on the weight or volume of the fodder mixture.

The prediction model for the second aspect can be stored on the electronic control unit, in particular in a storage device of or connected to the electronic control unit. The prediction model can be configured to predict a value for the fodder value based on previous generated data, in particular previous measurements. The prediction model can be trained based on previous generated data, in particular previous measurements, to improve the accuracy of the prediction of the fodder value. The previous measurements can include homogenously mixing at least two different fodder components, in particular in a laboratory, recording an optical spectra of the fodder mixture with a spectral sensor, measuring a fodder value with a sensor, in particular a sensor different from the spectral sensor, and forming a relation between the optical spectra and the measured fodder value. The prediction model can use the relation between the optical spectra and the measured fodder value to detect specific patterns in optical spectra. For example, the measured fodder value can correlate with an intensity of detected electromagnetic radiation for specific frequencies. The prediction model can be trained to detect specific patterns in newly presented optical spectra, i.e. optical spectra different from the training data, to predict or determine a fodder value based on the newly presented optical spectra. In other words, the prediction model can be trained by reference spectra to determine a fodder value based on an optical spectrum. For example, the electronic control unit can be configured to determine the amount of carbohydrate, fat, fiber, protein, dry matter and/or water of the fodder mixture in the fodder mixing apparatus based on one of the at least three optical spectra using the prediction model. This provides a relatively practical and quick determination of a fodder value. Thus, the prediction model can comprise a prediction model for carbohydrate, fat, fiber, protein, dry matter and/or water. The prediction model can also be trained with reference data from the literature and/or other sources.

Different prediction models may be used for carbohydrate, fat, fiber, protein, dry matter and/or water. It is also conceivable, however, that a prediction model is configured to determine two or more of carbohydrate, fat, fiber, protein, dry matter and/or water.

The prediction model can itself comprise a standard deviation for the predicted fodder value. The standard deviation for the predicted fodder value indicates an expected standard deviation for the predicted fodder value, in the case of a fully mixed fodder mixture. In other words, even if the fodder mixture is fully mixed, the fodder value may fluctuate around a mean value of the fodder value. The standard deviation for the predicted fodder value can take this fluctuation into account. For example, the prediction model can comprise a standard deviation for a predicted carbohydrate value, fat value, fiber value, protein value, dry matter value and/or water value. The standard deviation of the prediction model can be a weighted sum of the standard deviation for the predicted carbohydrate value, fat value, fiber value, protein value, dry matter value and/or water value. The standard deviation of the prediction model can be different from the calculated initial standard deviation of the determined fodder values. In particular, the calculated initial standard deviation of the determined fodder values will typically be larger than the standard deviation of the prediction model, since it includes the variations due to an incomplete mixing on top of the intrinsic model variations.

The fodder value for each of the at least three optical spectra can be of the same type, in particular selected from a group comprising carbohydrate, fat, fiber, protein, dry matter and water.

The determining of the current homogeneity parameter according to the second aspect can further comprise calculating, by the electronic control unit, a difference between the calculated initial standard deviation of the determined fodder values and an intrinsic error of the prediction model, wherein the current homogeneity parameter is determined based on the calculated difference. This allows determining a more accurate current homogeneity parameter. The intrinsic error of the prediction model can be based on random errors, systematic errors and/or other errors of the prediction model. The intrinsic error of the prediction model can be based on errors of the measuring instruments. The intrinsic error of the prediction model may particularly comprise or correspond to the standard deviation for the predicted fodder value as described herein above. By calculating a difference between the calculated initial standard deviation of the determined fodder values and an intrinsic error of the prediction model, variations of the fodder values due to uncertainties of the prediction model can be taken into account.

The method according to the second aspect can further comprise setting, by the electronic control unit, a value of a total homogeneity parameter to a predetermined value, in particular zero, when starting mixing and/or when adding further fodder to the mixing container, in particular a further fodder component, and updating, by the electronic control unit, the value of the total homogeneity parameter by calculating a weighted sum of the current homogeneity parameter and the set value of the total homogeneity parameter. The further fodder can be different from the fodder components of the fodder mixture. The further fodder can also be the same fodder component as one of the fodder components of the fodder mixture. The amount and/or type of further fodder can depend on one or more of the determined fodder values.

The method according to the second aspect can further comprise receiving, by the electronic control unit, at least one new optical spectrum, determining, by the electronic unit, a new fodder value for each of the at least one new optical spectra, calculating, by the electronic control unit, an updated standard deviation based on the new fodder value and previously determined fodder values, determining, by the electronic control unit, a new current homogeneity parameter based on the updated standard deviation, in particular a difference between the updated standard deviation and the intrinsic error of the prediction model, and updating, by the electronic control unit, the value of the total homogeneity parameter by calculating a weighted sum of the new current homogeneity parameter and the value of the total homogeneity parameter prior to the update. In this way, the development over time of the current homogeneity parameter can be taken into account. In addition, sudden changes of the current homogeneity parameter, in particular statistical outliers, can less affect the homogeneity determination. The at least one new optical spectrum may particularly be at least one further spectrum recorded by the spectral sensor.

The following may apply to both, the first and the second aspect of the disclosure.

The weights for the weighted sum for calculating the total homogeneity parameter can differ from each other or be equal to each other. The sum of the weights for the weighted sum can amount to a value of "1". For example, the weight for the total homogeneity parameter prior to the update can have a value of "0.35" and the weight for the new current homogeneity parameter can have a value of "0.65".

The optical spectra used for the described methods may particularly be obtained based on a sliding window having a width of at least three optical spectra and scanning a temporal sequence of optical spectra obtained by the spectral sensor. The increment for scanning the spectra with the sliding window may be 1. Thereby, overlapping windows may be obtained. In other words, two sets of at least three optical spectra may have optical spectra in common while differing from each other in at least one optical spectrum. The increment may also be any other integer, e.g. 2 or 3. Thus, also non-overlapping windows are possible.

The method according to the second aspect can further comprise determining, by the electronic control unit, a second fodder value for each of the at least three optical spectra using a second prediction model, wherein the second fodder value is in particular selected from a group comprising carbohydrate, fat, fiber, protein, dry matter and water, calculating, by the electronic control unit, an initial standard deviation of the determined second fodder values, and determining, by the electronic control unit, a second current homogeneity parameter based on the initial standard deviation of the determined second fodder values. The category of the second fodder value can be different from the category of the fodder value. For example, the second fodder value can be from the category "fat" and the fodder value can be from the category "fiber". In this case, the fodder values may also be referred to as "first fodder values".

By taking different fodder values into account, the accuracy of the homogeneity determination may be further improved.

The method can further comprise receiving, by the electronic control unit, at least one new optical spectrum, determining, by the electronic control unit, a new second fodder value for each of the at least one new optical spectra, calculating, by the electronic control unit, an updated standard deviation based on the new second fodder value and previously determined second fodder values, determining, by the electronic control unit, a new second current homogeneity parameter based on the updated standard deviation, in particular a difference between the updated standard deviation and the intrinsic error of the second prediction model, updating, by the electronic control unit, the value of the second total homogeneity parameter by calculating a weighted sum of the new second current homogeneity parameter and the value of a second total homogeneity parameter prior to the update, and calculating, by the electronic control unit, a weighted sum of the total homogeneity parameter and the second total homogeneity parameter. For the second total homogeneity parameter, the same considerations as described above for the total homogeneity parameter may apply. The at least one new optical spectrum may particularly be at least one further spectrum recorded by the spectral sensor. The at least one new optical spectrum for the new second fodder value and the at least one new optical spectrum for the new fodder value can be identical.

The method according to the second aspect can further comprise normalizing the current homogeneity parameter and/or the second current homogeneity parameter, in particular by forming a ratio of the standard deviation of the prediction model for the fodder value and the current homogeneity parameter and/or by forming a ratio of the standard deviation of the second prediction model for the second fodder value and the second current homogeneity parameter. The standard deviation of the prediction model for the fodder value can be based on previous measurements, in particular on optical spectra from a fully mixed fodder mixture as detailed herein above. By normalizing the current homogeneity parameter and/or the second current homogeneity parameter, the parameters may particularly be mapped to the interval from 0 to 1. A value of "0" of the ratio can mean that the fodder mixture is not mixed at all and a value of "1" can mean that the fodder mixture is fully mixed.

The method can further comprise calculating, by the electronic control unit, a difference between the calculated initial standard deviation of the determined second fodder values and an intrinsic error of the second prediction model, wherein the second current homogeneity parameter is determined based on the calculated difference. This allows calculating a more accurate second current homogeneity parameter.

The method can further comprise calculating an average current homogeneity parameter based on the current homogeneity parameter and the second current homogeneity parameter, in particular wherein updating, by the electronic control unit, the value of the total homogeneity parameter is performed by calculating a weighted sum of the average current homogeneity parameter and the set value of the total homogeneity parameter.

The method can further comprise setting, by the electronic control unit, a value of a second total homogeneity parameter to a predetermined value, in particular zero, when starting mixing and/or when adding further fodder to the mixing container, in particular a further fodder component, updating, by the electronic control unit, the value of the second total homogeneity parameter by calculating a weighted sum of the second current homogeneity parameter and the set value of the second total homogeneity parameter, and calculating, by the electronic control unit, a weighted sum of the total homogeneity parameter and the second total homogeneity parameter. The weighted sum of the total homogeneity parameter and the second total homogeneity parameter may be set as new total homogeneity parameter. The new total homogeneity parameter may be a more accurate estimate for the homogeneity of the fodder mixture. The amount and/or type of further fodder can depend on one or more of the determined fodder values and/or on one or more of the determined second fodder values.

The disclosure according to a third aspect provides a combination of the method according to the first aspect and the method according to the second aspect. In particular, the disclosure according to the third aspect provides a method for determining a current homogeneity parameter, wherein the current homogeneity parameter is determined by calculating a weighted sum of a first current homogeneity parameter and a second current homogeneity parameter, wherein the first current homogeneity parameter is determined by the method according to the first aspect and the second current homogeneity parameter is determined by the method according to the second aspect. This provides a more accurate determination of the current homogeneity parameter. The at least three optical spectra of the method of the first aspect can be the same as the at least three optical spectra of the method of the second aspect.

The following may apply to all three aspects of the disclosure.

Determining a current homogeneity parameter according to one of the described methods may be initiated manually or automatically. The fodder mixing apparatus may particularly start the determination automatically once all fodder components have been added to the mixing container. The fodder mixing apparatus may determine that all fodder components have been loaded based on input by the farmer and/or based on sensor data.

Alternatively or additionally, the fodder mixing apparatus may initiate the determination of a current homogeneity parameter automatically at or after predefined steps of the loading process, i.e. during the process of adding fodder components to the mixing container. The fodder mixing apparatus, for example, may start the determination automatically after a predetermined number of fodder components of a ration have been added to the mixing container. The loading process, in particular a desired amount of each fodder component and a loading sequence of the fodder components, may be programmable by the farmer. In particular, the farmer may define states in the loading process when the current homogeneity parameter is to be determined. The fodder mixing apparatus may determine whether a defined process state has been reached based on input by the farmer and/or based on sensor data, in particular based on data of a weight sensor.

The mixing of the fodder mixture, in particular of the at least two different fodder components, can be stopped manually or automatically, when the total homogeneity parameter or the weighted sum of the total homogeneity parameter and the second total homogeneity parameter meets a predetermined criterion, in particular, when a predetermined value for the total homogeneity parameter or the weighted sum of the total homogeneity parameter and the second total homogeneity parameter is reached or exceeded. The predetermined value may, for instance, be in a range of 0.90 to 0.95 for total homogeneity parameters ranging from 0 to 1. The methods, thus, may further comprise the step of determining whether the total homogeneity parameter or the weighted sum of the total homogeneity parameter and the second total homogeneity parameter meets a predetermined criterion.

The methods described above can further comprise determining a rate-of-change of the total homogeneity parameter or the weighted sum of the total homogeneity parameter and the second total homogeneity parameter and estimating a remaining mixing time for the mixing process. The remaining mixing time can be a time until the total homogeneity parameter or the weighted sum of the total homogeneity parameter and the second total homogeneity parameter meets the predetermined criterion as described above.

The mixing of the fodder mixture can be stopped manually or automatically, when the predetermined criterion is met as described above. The remaining mixing time can be estimated by taking the value and the rate-of-change of the total homogeneity parameter or the weighted sum of the total homogeneity parameter and the second total homogeneity parameter and the predetermined criterion into account. The remaining mixing time can also be estimated by taking historical data into account, for example, the mixing time from previous mixing processes, in particular, the mixing time until the predetermined criterion is met. The estimated remaining mixing time for the mixing process can be displayed to an operator of the fodder mixing apparatus, for example, on a Human-Machine Interface. In addition or as alternative, the total homogeneity parameter and/or the weighted sum of the total homogeneity parameter and the second total homogeneity parameter and/or the current homogeneity parameter can be displayed to the operator, in particular, on the Human-Machine Interface. The fodder mixing apparatus can comprise the Human-Machine Interface.

The fodder mixing apparatus can further comprise a storage medium. Data generated by the methods described above, can be stored on the storage medium, in particular, data that has been received, determined, calculated, updated, formed and/or set, in particular by the electronic control unit, such as optical spectra, current homogeneity parameters, total homogeneity parameters, fodder values, standard deviations, intrinsic errors of prediction models and/ or weighted sums. The stored data can be displayed by a Human-Machine Interface, which can be comprised by the fodder mixing apparatus.

This has the advantage that historic data can be viewed, for example to ensure consistent quality of the fodder mixture. For example, the mixing of the fodder mixture can be stopped when a predetermined criterion is met, and, in particular current fodder values can be displayed to the operator. The operator can analyze and/or validate the fodder values, in particular by comparing the fodder values to historic fodder values.

The methods described above can further comprise that further fodder, in particular a further fodder component, or water is added, when the total homogeneity parameter or the weighted sum of the total homogeneity parameter and the second total homogeneity parameter meets a predetermined criterion, in particular, when a predetermined value for the total homogeneity parameter or the weighted sum of the total homogeneity parameter and the second total homogeneity parameter is reached or exceeded.

It can be necessary to add further fodder or water to the at least two different fodder components in a later stage of the mixing process, for example, if it is necessary or advantageous to mix the at least two different fodder components first. The embodiments described above allow a more accurate determination of a time for the addition of the further fodder or water. The further fodder or water may be added while mixing the fodder mixture or after stopping mixing, in particular temporarily.

The two fodder components can differ in at least one characteristic, for example, in the amount of carbohydrates, fat, fiber, protein, dry matter, water, particle size and/or temperature. At least one of the two fodder components can be of a group including corn, grain, hay, silage, and/or straw. The fodder mixture can be mixed, for example, for cattle, chicken, pigs, sheep, horses, goats, camels, buffalos and/or ducks. The fodder mixture can also comprise at least one fodder component and water, in particular at least two fodder components and water. This allows to increase the moisture of the at least one fodder component.

Adding further fodder to the mixing container can decrease the current homogeneity parameter determined after adding the further fodder. This effect can be delayed, in particular time-delayed, depending, in particular, on where the spectral sensor is arranged, because it can require a certain amount of mixing of the fodder mixture until the further fodder is recorded by the spectral sensor. The described methods may, thus, comprise continuing mixing after adding further fodder for a predetermined amount of time even if the total homogeneity parameter or the weighted sum of the total homogeneity parameter and the second total homogeneity parameter meet a predetermined criterion. In other words, even if the mixing could otherwise be stopped, the mixing continues after adding further fodder for a predetermined time so that a decrease in the homogeneity could be detected. Alternatively or additionally, a new predetermined criterion, in particular a new threshold value, may be set or used.

Subsequently determined current homogeneity parameters can increase again with further mixing of the fodder mixture, in particular to a value before adding the further fodder. The time and/or mixing of the fodder mixture required to reach the same value of the current homogeneity parameter can depend on different parameters, in particular on the amount of further fodder added in relation to the amount of fodder mixture already in the mixing container and/or on how well the further fodder can be homogeneously mixed into the fodder mixture, for example, heavily clumped further fodder can be more difficult to homogeneously mix into the fodder mixture, as the clumps have to be broken up first. The predetermined time for which mixing continues after adding further fodder may, thus, depend on the type of fodder.

The fodder mixing apparatus can be a towed or self-propelled fodder mixer wagon, a fodder robot or a stationary mixing plant. The mixing container of the fodder mixing apparatus can comprise a bottom and a wall extending upwards from the bottom. The mixing container can comprise at least one mixing auger rotatably arranged in the mixing container.

The fodder mixing apparatus can comprise the electronic control unit. The electronic control unit can comprise an interface for the spectral sensor, in particular for receiving optical spectra. The electronic control unit can comprise a processing unit. The processing unit can comprise a processor. The electronic control unit may further comprise or be connected to a data storage unit. The processing unit can receive the optical spectra for determining the current homogeneity parameter of the fodder mixture via the interface to the spectral sensor and may store the optical spectra in the data storage unit.

The disclosure further provides a fodder mixing apparatus that comprises a mixing container, at least one mixing device, in particular a mixing auger, arranged in the mixing container, a spectral sensor, and an electronic control unit, wherein the fodder mixing apparatus, in particular the electronic control unit, is configured to carry out at least one of the previously described methods.

The fodder mixing apparatus can comprise a Human-Machine Interface for displaying, in particular to an operator, the remaining mixing time as estimated according to one of the methods described above and/or the total homogeneity parameter and/or the weighted sum of the total homogeneity parameter and the second total homogeneity parameter and/or the current homogeneity parameter.

The fodder mixing apparatus can comprise a weighing computer, wherein the weighing computer can determine the weight of the fodder mixture. The fodder mixing apparatus can be configured to automatically load the fodder mixture in the mixing container, in particular until a predetermined weight of the fodder mixture is reached or exceeded. The fodder mixing apparatus, in particular the electronic control unit, can be configured to carry out, in particular automatically, at least one of the previously described methods after a predetermined weight of the fodder mixture is reached or exceeded. In this way, the fodder mixture can be prepared in a streamlined manner.

The electronic control unit may have one or more features described herein above in the context of the disclosed methods.

The disclosure further provides a computer program product comprising instructions, which, when the program is executed by a computer, cause the computer to carry out the steps of at least one of the previously described methods.

Embodiments of the invention will now be described in combination with the enclosed figures.

### Brief description of the figures:

- Figure 1: Fodder mixing apparatus with a spectral sensor arranged on a mixing device;
- Figure 2: Exemplary plot of an optical spectrum;
- Figure 3: Exemplary schematic illustration of a method;
- Figure 4: Exemplary plot of values of a total homogeneity parameter over a number of updates of the total homogeneity parameter;
- Figure 5: Further exemplary schematic illustration of a method; and
- Figure 6: Exemplary plot of a development of a weighted sum of a total homogeneity parameter and a second total homogeneity parameter.

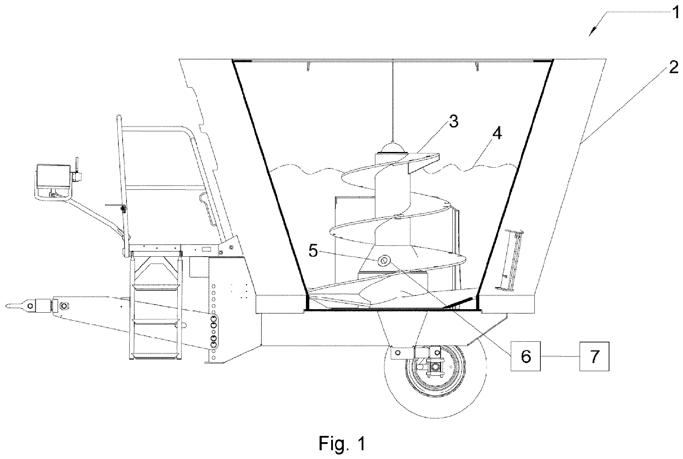

Figure 1 shows a fodder mixing apparatus 1, which is configured as a fodder mixing wagon. The fodder mixing apparatus 1 comprises a mixing container 2, which is open at the top. A mixing device 3, which is configured as a mixing auger, is rotatable arranged in the mixing container 2. A fodder mixture 4, comprising at least two fodder components, is loaded into the mixing container 2 via the top opening of the mixing container 2 and mixed by the mixing device 3 in such a way that the fodder mixture 4 is conveyed upwards and flows back to the bottom of the mixing container 2.

A spectral sensor 5, which is configured as a NIR-sensor, is arranged on the mixing device 3 and records optical spectra 6 of the fodder mixture in a temporal sequence. For the measurement, the spectral sensor 5 probes the fodder mixture 4 in front of the spectral sensor 5, by emitting electromagnetic radiation and receiving the electromagnetic radiation reflected by the fodder mixture 4. The frequency of the electromagnetic radiation received by the spectral sensor 5 can, for example, range from 1300 nm to 2525 nm.

The fodder mixing apparatus 1 further comprises an electronic control unit 7, which receives the optical spectra 6 via a data connection from the spectral sensor 5.

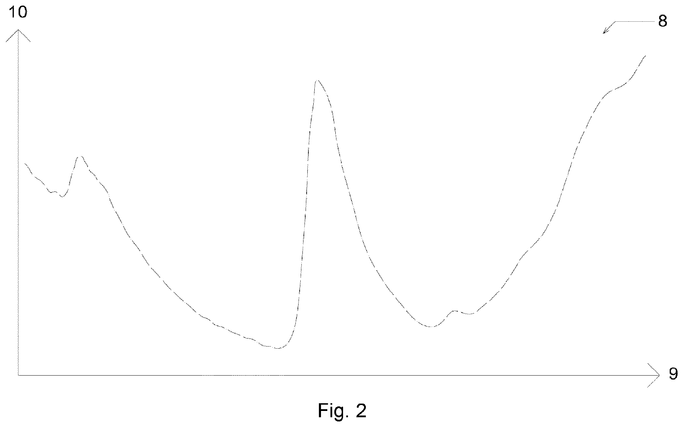

Figure 2 shows an exemplary plot 8 of an optical spectrum 6 with a frequency of the electromagnetic radiation 9, which is detected by a spectral sensor 5, in nanometers on the x-axis and a corresponding detected intensity 10 of the electromagnetic radiation in arbitrary units on the y-axis. The frequency 9 ranges in this example from 1300 nm to 2525 nm. The optical spectrum 6 has been recorded by a spectral sensor 5 with a spectral resolution of 5 nm. Thus, a value for the detected intensity 10 is determined for each range of 5 nm in the frequency range 9 of the electromagnetic radiation.

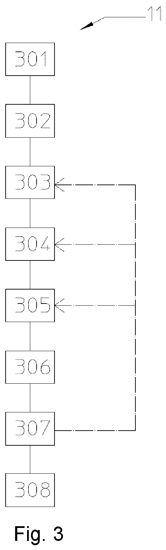

Figure 3 shows in a schematic illustration an exemplary method 11 for determining a current homogeneity parameter of a fodder mixture 4 for farm animals while mixing the fodder mixture 4 in a mixing container 2 of a fodder mixing apparatus 1. The individual steps are not necessarily performed in this temporal order. Unless a certain step requires the output of a previous step as input, the steps may also be performed in a different order and/or at least partially overlapping.

In a first step 301, when starting the mixing of the fodder mixture 4, an electronic control unit 7 sets a total homogeneity parameter to a predetermined value, in this example a value of "0".

In a second step 302, a spectral sensor 5 records, in sequence, optical spectra 6 of the fodder mixture 4 while mixing.

In a third step 303, the electronic control unit 7 receives three optical spectra 6. In this case, the three optical spectra 6 have been recorded in succession, i.e. directly one after the other. The three optical spectra 6 may be received in sequence as they are recorded by spectral sensor 5 or may be received as a batch from the spectral sensor 5.

In a next step 304, the electronic control unit 7 calculates a correlation between the three optical spectra 6. In this example, the electronic control unit 7 calculates a correlation based on Lin's concordance correlation coefficient, which ranges from a value "0" to a value "1". The correlation is calculated pairwise, i.e. the coefficient is calculated for every pair of two optical spectra of the three optical spectra and a mean value of the calculated coefficients is determined. Thus, three coefficients are calculated for the three optical spectra and a mean value of the three calculated coefficients is determined. The first coefficient is calculated based on the first and second optical spectrum, the second coefficient based on the first and third optical spectrum and the third coefficient based on the second and third optical spectrum. The mean value of the calculated coefficients is then set as Lin's concordance correlation coefficient for the three optical spectra.

In a next step 305, the electronic control unit 7 determines the current homogeneity parameter based on Lin's concordance correlation coefficient. In this example, the current homogeneity parameter corresponds to Lin's concordance correlation coefficient, i.e. the mean value of the three calculated coefficients. In this example, a value of "0" means that the fodder mixture is not mixed and a value of "1" means that the fodder mixture is homogenously mixed.

In a step 306, the electronic control unit 7 updates the value of the total homogeneity parameter by calculating a weighted sum of the current homogeneity parameter as determined in step 305 and the set value of the total homogeneity parameter.

In a particular example, the total homogeneity parameter may be calculated by a sum of the set value of the total homogeneity parameter of "0" weighted by a weight of "0.35" and an exemplary current homogeneity parameter of "0.4" weighted by a weight of "0.65". Calculating the weighted sum leads in this example to a value for the total homogeneity parameter of "0.26". In view of the weighted sum, the total homogeneity parameter varies less in time than the current homogeneity parameter, which may be influenced by statistical variations of the optical spectra 6. Thus, sudden changes of the current homogeneity parameter, for instance statistical outliers, may be smoothened by the weighted sum. The total homogeneity parameter and its temporal evolution, thus, may provide a more accurate measure for the homogeneity.

In a step 307, the electronic control unit 7 repeatedly determines a new current homogeneity parameter by carrying out steps 303, 304 and 305 based on new optical spectra recorded by the spectral sensor. The electronic control unit 7 updates the value of the total homogeneity parameter after each determination of a new current homogeneity parameter by calculating a weighted sum of the new current homogeneity parameter and the value of the total homogeneity parameter prior to the update. For each updated value of the total homogeneity parameter, the electronic control unit 7 may check whether the updated value of the total homogeneity parameter meets a predetermined criterion, for instance whether the total homogeneity parameter exceeds a threshold value T.

In a last step 308, the mixing of the at least two different fodder components is stopped automatically, when the total homogeneity parameter meets the predetermined criterion. In this embodiment, the mixing is stopped, when the total homogeneity parameter exceeds a threshold value T with a value of "0.9".

In an example, further fodder, in particular a further fodder component, may be added to the mixing container 2 after stopping the mixing process. Stopping the mixing process for adding the further fodder component is, however, optional. When the further fodder is added to the mixing container 2, the electronic control unit 7 may set the value for the total homogeneity parameter to a predetermined value, in this example a value of "0". Thereafter, the method can be repeated from step 302.

Alternatively, the total homogeneity parameter may be set to or maintained at the value prior to adding the further fodder, but mixing continues for a predetermined time, despite the total homogeneity parameter meeting the predetermined criterion. The predetermined time may be set based on empirical data. During the continued mixing, the described method may particularly revert to step 307 described herein. In view of the added fodder, the total homogeneity parameter will first decrease within the predetermined time and thereafter, as mixing continues, increase again. After the predetermined time, mixing may again be stopped, in particular automatically, if the total homogeneity parameter meets the predetermined criterion or a different predetermined criterion, e.g. using a different threshold value T'.

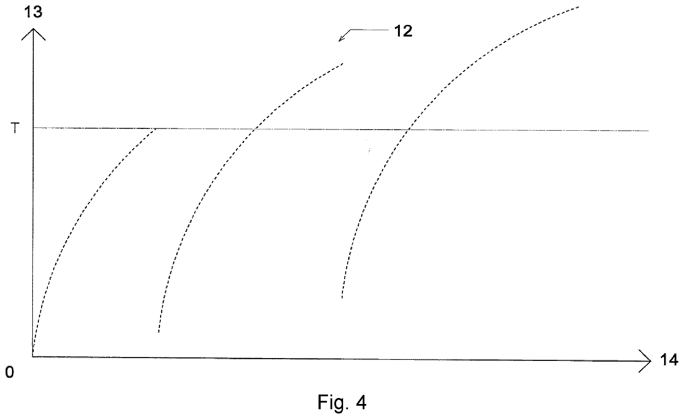

Figure 4 shows an exemplary plot 12 of a development of a value of total homogeneity parameter while mixing and updating the value of the total homogeneity parameter. The value of the total homogeneity parameter is on the y-axis and the number of updates 14 of the total homogeneity parameter is on the x-axis. The number of updates 14 of the total homogeneity parameter starts at a value of "0" and increases by a value of "1" with every update of the total homogeneity parameter. A dashed line parallel to the x-axis illustrates a threshold value T for the value of the total homogeneity parameter with an exemplary value of "0.5".

In a first curve on the left side of plot 12, the plot 12 shows that the value of the total homogeneity parameter increases until the threshold value T is reached. In this example, when the threshold value T is reached, further fodder is added to the mixing container 2.

In a second curve in the middle of plot 12, similar to the first curve of plot 12, the value of the total homogeneity parameter increases until a second threshold value is reached. Since further fodder has been added to the mixing container 2, in this example, the values of the total homogeneity parameters in the beginning of the second curve of plot 12 are lower than at the end of the first curve of plot 12. A similar development of the value of the total homogeneity parameter is shown in a third curve on the right side of plot 12, after again further fodder was added to the mixing container 2. In this example, the values of the total homogeneity parameter in the beginning of the third curve of plot 12 are higher than in the second curve of plot 12, because the ratio of the further added fodder in relation to the fodder mixture 4 already in the mixing container 2 is smaller in the third curve compared to the second curve of plot 12. In other words, the derived homogeneity of the fodder mixture 4 is higher in the beginning, because less additional fodder is added in relation to the fodder mixture 4 already in the mixing container 2.

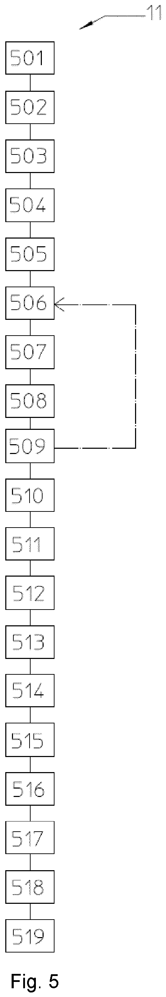

Figure 5 shows in a schematic illustration a further method 11 for determining a current homogeneity parameter of the fodder mixture 4 for farm animals while mixing the fodder mixture 4 in a mixing container 2 of a fodder mixing apparatus 1. Again, the individual steps are not necessarily performed in this temporal order. Unless a certain step requires the output of a previous step as input, the steps may also be performed in a different order and/or at least partially overlapping.

In a first step 501, when starting the mixing of the fodder mixture 4, an electronic control unit 7 sets a total homogeneity parameter and a second total homogeneity parameter to a predetermined value, in this example a value of "0".

In a second step 502, a spectral sensor 5 records, in sequence, optical spectra 6 of the fodder mixture 4 while mixing.

In a third step 503, the electronic control unit 7 receives at least three optical spectra 6. In this case, the electronic control unit 7 receives three optical spectra 6, which have been recorded in succession, i.e. directly one after the other. The three optical spectra 6 may be received in sequence as they are recorded by spectral sensor 5 or may be received as a batch from the spectral sensor 5.

In a next step 504, the electronic control unit 7 determines a fodder value for each of the three optical spectra 6 using a prediction model. In an example, the fodder values may be of the category carbohydrate, fat, fiber, protein, dry matter or water. In this specific example the fodder value is of the category "protein". Thus, the fodder values are referred to as "protein 1", "protein 2" and "protein 3" in the following. As detailed further below, fodder values for one or more further categories may be taken into account. The prediction model is stored on the electronic control unit 7. In this example, the prediction model has been trained by reference spectra from previous measurements, which have been carried out in a laboratory. Creating and training the prediction model, thus, is not necessarily part of the described method.

In a step 507, the electronic control unit 7 calculates an initial standard deviation of the determined fodder values, i.e. the set comprising the values "protein 1", "protein 2" and "protein 3".

Then, the electronic control unit 7 calculates a difference between the calculated initial standard deviation of the determined fodder values and an intrinsic error of the prediction model, wherein the current homogeneity parameter is determined based on the calculated difference. In particular, the current homogeneity parameter may be determined or set to correspond to the calculated difference.

The current homogeneity parameter may be normalized by forming a ratio of a standard deviation of the prediction model for the fodder value, i.e. for the fodder value of the category "protein", and the current homogeneity parameter thus determined.

In a next step 506, the electronic control unit 7 updates the value of the total homogeneity parameter, which has been set to a value of "0" in step 501, by calculating a weighted sum of the current homogeneity parameter as determined in step 506 and the set value of the total homogeneity parameter.

In an optional step 507, the electronic control unit 7 determines a second fodder value for each of the three optical spectra, which have been received in step 503, using a second prediction model. The second fodder values are in this example of the category "fiber" and are referred to as "fiber 1", "fiber 2" and "fiber 3" in the following.

In a step 508, the electronic control unit 7 calculates an initial standard deviation of the determined second fodder values, i.e. the set comprising the values "fiber 1", "fiber 2" and "fiber 3". Then, the electronic control unit 7 calculates a difference between the calculated initial standard deviation of the determined second fodder values and an intrinsic error of the second prediction model, wherein the second current homogeneity parameter is determined based on the calculated difference. In particular, the second current homogeneity parameter may be determined or set to correspond to the calculated difference.

The second current homogeneity parameter may be normalized by forming a ratio of a standard deviation of the second prediction model for the second fodder value, i.e. for the second fodder value of the category "fiber" and the second current homogeneity parameter thus determined.

In a step 509, the electronic control unit 7 updates the value of the second total homogeneity parameter, which has been set to a value of "0" in step 501, by calculating a weighted sum of the second current homogeneity parameter and the set value of the second total homogeneity parameter.

Then, the electronic control unit 7 calculates a weighted sum of the total homogeneity parameter determined in step 506 and the second total homogeneity parameter determined in step 509. In this embodiment, since the parameters have been normalized, the value of the weighted sum ranges from a value of "0" to a value of "1", wherein a value of "0" means that the fodder mixture 4 is not mixed at all and a value of "+1" means that the fodder mixture 4 is homogenously mixed.

In a step 510, the electronic control unit 7 receives at least one new optical spectrum 6. In this example, the electronic control unit 7 receives one new optical spectrum, which has been recorded after the three optical spectra 6, which have been received in step 503.

In a step 511, the electronic control unit 7 determines a new fodder value for the new optical spectrum 6, which is referred to as "protein 4" in the following.

In a step 512, the electronic control unit 7 calculates an updated standard deviation based on the new fodder value, i.e. based on "protein 4", and previously determined fodder values, in this case, the fodder values "protein 1", "protein 2" and "protein 3".

Then, the electronic control unit 7 determines a new current homogeneity parameter based on a difference between the updated standard deviation and the intrinsic error of the prediction model.

The new current homogeneity parameter is normalized by forming a ratio of the updated standard deviation and the new current homogeneity parameter.

In a step 513, the electronic control unit 7 updates the value of the total homogeneity parameter by calculating a weighted sum of the new current homogeneity parameter and the value of the total homogeneity parameter prior to the update.

In a step 514, the electronic control unit 7 receives at least one new optical spectrum 6. In this example, the electronic control unit 7 receives one new optical spectrum, which has been recorded after the three optical spectra 6, which have been received in step 503. The new optical spectrum 6 may be the same new optical spectrum 6 used in step 511.

In a step 515, the electronic control unit 7 determines a new second fodder value for the new optical spectrum 6, which is referred to as "fiber 4" in the following.

In a step 516, the electronic control unit 7 calculates an updated standard deviation based on the new second fodder value, i.e. based on "fiber 4", and previously determined second fodder values, in this case, the second fodder values "fiber 1", "fiber 2" and "fiber 3".

Then, the electronic control unit 7 determines a new second current homogeneity parameter based on a difference between the updated standard deviation and the intrinsic error of the second prediction model.

The new second current homogeneity parameter is normalized by forming a ratio of the updated standard deviation and the new second current homogeneity parameter.

In a step 517, the electronic control unit 7 updates the value of the second total homogeneity parameter by calculating a weighted sum of the new second current homogeneity parameter and the value of the second total homogeneity parameter prior to the update. Then, the electronic control unit 7 calculates a weighted sum of the total homogeneity parameter and the second total homogeneity parameter. The electronic control unit 7 may then check whether the weighted sum of the total homogeneity parameter and the second total homogeneity parameter meets a predetermined criterion, for instance whether the weighted sum of the total homogeneity parameter and the second total homogeneity parameter exceeds a threshold value T.

In a step 518, the mixing of the at least two different fodder components is stopped automatically, when the weighted sum of the total homogeneity parameter and the second total homogeneity parameter meets the predetermined criterion. The method may further comprise determining the fodder value and the second fodder value for the fodder mixture after stop of the mixing, particularly as described herein above. The thus obtained final fodder value and the final second fodder value may be displayed on a Human-Machine Interface of the fodder mixing apparatus.

In a last step 519, which is also optional, further fodder is added and the total homogeneity parameter and the second total homogeneity parameter are set again to a predetermined value, for example a value of "0". Thereafter, the method can be repeated from step 502.

The amount and/or type of further fodder can depend on one or more of the determined fodder values and/or on one or more of the determined second fodder values. In particular, the amount and/or type of further fodder may be determined based on a difference between one or more determined fodder values and one or more desired values for the respective fodder values and/or on a difference between one or more determined second fodder values and one or more desired values for the respective second fodder values.

Alternatively, the total homogeneity parameter and the second total homogeneity parameter may be set to or maintained at the values prior to adding the further fodder, but mixing continues for a predetermined time, despite the weighted sum of the total homogeneity parameter and the second total homogeneity parameter meeting the predetermined criterion. The predetermined time may be set based on empirical data. In view of the added fodder, the total homogeneity parameter and the second total homogeneity parameter will first decrease within the predetermined time and thereafter, as mixing continues, increase again. After the predetermined time, mixing may again be stopped, in particular automatically, if the weighted sum of the total homogeneity parameter and the second total homogeneity parameter meets the predetermined criterion or a different predetermined criterion, e.g. using a different threshold value T'.

The further fodder may particularly be added if the final fodder value and/or the final second fodder value do not correspond to desired or predetermined values. Stopping the mixing process for adding the further fodder component is optional.

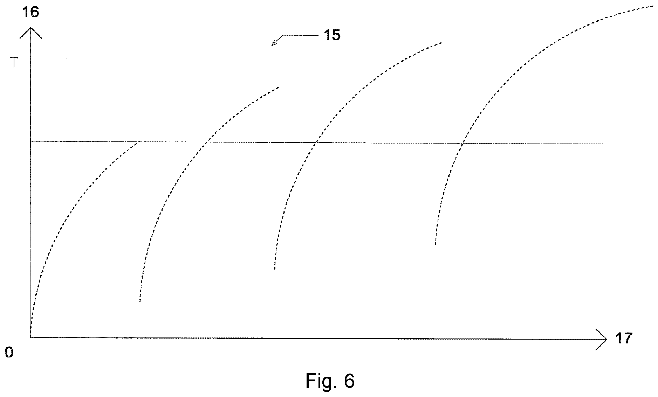

Figure 6 shows an exemplary plot 15 of a development of a value of a weighted sum of a total homogeneity parameter and a second total homogeneity parameter while mixing and repeatedly calculating the weighted sum. A value for the weighted sum 16 is on the y-axis and a number 17 of calculated weighted sums is on the x-axis. A dashed line parallel to the x-axis illustrates a threshold value T of the weighted sum with an exemplary value of "0.5".

The plot 15 is visually divided into four curves, wherein each curve shows the value of the weighted sum increasing until a threshold value is reached (only one exemplary threshold value T is illustrated for the first curve of plot 15).

When one of the four curves reaches its threshold value T, further fodder is added in the mixing container 2, which lowers the values of the weighed sum in the beginning of the second to fourth curve of plot 15 in this example.

Figure 6 also shows that each curve, when viewed from left to right, starts out at a higher value of the weighted sum and has a steeper slope compared to the previous curve, meaning that it takes less calculations of the weighted sum and thus, requires less mixing of the fodder mixture 4, to reach the same or around the same value of the weighted sum after adding the further fodder. In this example, this is in particular because less additional fodder is added in relation to the fodder mixture already in the mixing container 2 with each subsequent addition of further fodder.

### List of reference signs

- 1: fodder mixing apparatus
- 2: mixing container
- 3: mixing device
- 4: fodder mixture
- 5: spectral sensor
- 6: optical spectra
- 7: electronic control unit
- 8: plot of an optical spectrum
- 9: by a spectral sensor detected frequency of an electromagnetic radiation
- 10: by a spectral sensor detected intensity of an electromagnetic radiation
- 11: method for determining a current homogeneity parameter of a fodder mixture
- 12: plot of a development of a total homogeneity parameter
- 13: value of a total homogeneity parameter
- 14: number of updates of a total homogeneity parameter
- 15: plot of a development of a weighted sum of a total homogeneity parameter and a second total homogeneity parameter
- 16: value for a weighted sum of a total homogeneity parameter and a second total homogeneity parameter
- 17: number of calculated weighted sums of a total homogeneity parameter and a second total homogeneity parameter
- T: threshold value

## Claims

1. Method (11) for determining a current homogeneity parameter of a fodder mixture (4) for farm animals while mixing the fodder mixture (4) in a mixing container (2) of a fodder mixing apparatus (1), wherein the fodder mixture (4) comprises at least two different fodder components, comprising:
- recording, in sequence, optical spectra (6) of the fodder mixture (4) with a spectral sensor (5) while mixing,
**characterized by the steps:**
a) receiving, by an electronic control unit (7), at least three optical spectra (6) recorded by the spectral sensor (5),
b) calculating, by the electronic control unit (7), a correlation between the at least three optical spectra (6), and
c) determining, by the electronic control unit (7), the current homogeneity parameter based on the correlation between the at least three optical spectra (6).

2. The method according to claim 1, wherein the correlation is a linear correlation, in particular a Pearson correlation, or based on Lin's concordance correlation coefficient.

3. The method according to claim 1 or claim 2, further comprising the steps of:
- setting, by the electronic control unit, a value of a total homogeneity parameter to a predetermined value, in particular zero, when starting mixing and/or when adding further fodder to the mixing container, in particular a further fodder component, and
- updating, by the electronic control unit, the value of the total homogeneity parameter by calculating a weighted sum of the current homogeneity parameter and the set value of the total homogeneity parameter.

4. The method according to claim 3, further comprising:
- repeatedly determining, by the electronic control unit, a new current homogeneity parameter by carrying out steps a) to c) based on different optical spectra, and
- updating, by the electronic control unit, the value of the total homogeneity parameter after each determination of a new current homogeneity parameter by calculating a weighted sum of the new current homogeneity parameter and the value of the total homogeneity parameter prior to the update.

5. Method (11) for determining a current homogeneity parameter of a fodder mixture (4) for farm animals while mixing the fodder mixture (4) in a mixing container (2) of a fodder mixing apparatus (1), wherein the fodder mixture (4) comprises at least two different fodder components, comprising:
- recording, in sequence, optical spectra (6) of the fodder mixture (4) with a spectral sensor (5) while mixing,
**characterized by the steps:**
- receiving, by an electronic control unit (7), at least three optical spectra (6) recorded by the spectral sensor (5),
- determining, by the electronic control unit (7), a fodder value for each of the at least three optical spectra (6) using a prediction model, wherein the fodder values are in particular selected from a group comprising carbohydrate, fat, fiber, protein, dry matter and water,
- calculating, by the electronic control unit (7), an initial standard deviation of the determined fodder values, and
- determining, by the electronic control unit (7), the current homogeneity parameter based on the initial standard deviation of the determined fodder values.

6. The method according to claim 5, wherein determining the current homogeneity parameter further comprises:
- calculating, by the electronic control unit, a difference between the calculated initial standard deviation of the determined fodder values and an intrinsic error of the prediction model, wherein the current homogeneity parameter is determined based on the calculated difference.

7. The method according to claim 5 or claim 6, further comprising:
- setting, by the electronic control unit, a value of a total homogeneity parameter to a predetermined value, in particular zero, when starting mixing and/or when adding further fodder to the mixing container, in particular a further fodder component, and
- updating, by the electronic control unit, the value of the total homogeneity parameter by calculating a weighted sum of the current homogeneity parameter and the set value of the total homogeneity parameter.

8. The method according to claim 7, further comprising:
- receiving, by the electronic control unit, at least one new optical spectrum,
- determining, by the electronic control unit, a new fodder value for each of the at least one new optical spectra,
- calculating, by the electronic control unit, an updated standard deviation based on the new fodder value and previously determined fodder values,
- determining, by the electronic control unit, a new current homogeneity parameter based on the updated standard deviation, in particular a difference between the updated standard deviation and the intrinsic error of the prediction model, and
- updating, by the electronic control unit, the value of the total homogeneity parameter by calculating a weighted sum of the new current homogeneity parameter and the value of the total homogeneity parameter prior to the update.

9. The method according to any one of claims 5 to 8, further comprising:
- determining, by the electronic control unit, a second fodder value for each of the at least three optical spectra using a second prediction model, wherein the second fodder values are in particular selected from a group comprising carbohydrate, fat, fiber, protein, dry matter and water,
- calculating, by the electronic control unit, an initial standard deviation of the determined second fodder values, and
- determining, by the electronic control unit, a second current homogeneity parameter based on the initial standard deviation of the determined second fodder values.

10. The method according to any one of claims 5 to 9, wherein the method further comprises normalizing the current homogeneity parameter and/or the second current homogeneity parameter, in particular by forming a ratio of the standard deviation of the prediction model for the fodder value and the current homogeneity parameter and/or by forming a ratio of the standard deviation of the second prediction model for the second fodder value and the second current homogeneity parameter.

11. The method according to claim 9 or claim 10, further comprising calculating, by the electronic control unit, a difference between the calculated initial standard deviation of the determined second fodder values and an intrinsic error of the second prediction model, wherein the second current homogeneity parameter is determined based on the calculated difference.

12. The method according to any one of claims 9 to 11, further comprising calculating an average current homogeneity parameter based on the current homogeneity parameter and the second current homogeneity parameter, in particular wherein updating, by the electronic control unit, the value of the total homogeneity parameter is performed by calculating a weighted sum of the average current homogeneity parameter and the set value of the total homogeneity parameter.

13. The method according to any one of claims 9 to 12, further comprising:
- setting, by the electronic control unit, a value of a second total homogeneity parameter to a predetermined value, in particular zero, when starting mixing and/or when adding further fodder to the mixing container, in particular a further fodder component,
- updating, by the electronic control unit, the value of the second total homogeneity parameter by calculating a weighted sum of the second current homogeneity parameter and the set value of the second total homogeneity parameter, and
- calculating, by the electronic control unit, a weighted sum of the total homogeneity parameter and the second total homogeneity parameter.

14. The method according to claim 13, further comprising:
- receiving, by the electronic control unit, at least one new optical spectrum,
- determining, by the electronic control unit, a new second fodder value for each of the at least one new optical spectra,
- calculating, by the electronic control unit, an updated standard deviation based on the new second fodder value and previously determined second fodder values,
- determining, by the electronic control unit, a new second current homogeneity parameter based on the updated standard deviation, in particular a difference between the updated standard deviation and the intrinsic error of the second prediction model,
- updating, by the electronic control unit, the value of the second total homogeneity parameter by calculating a weighted sum of the new second current homogeneity parameter and the value of the second total homogeneity parameter prior to the update, and
- calculating, by the electronic control unit, a weighted sum of the total homogeneity parameter and the second total homogeneity parameter.

15. Fodder mixing apparatus (1) comprising:
- a mixing container (2),
- at least one mixing device (3), in particular a mixing auger (3), arranged in the mixing container (2),
- a spectral sensor (5), and
- an electronic control unit (7),
wherein the fodder mixing apparatus, in particular the electronic control unit (7), is configured to carry out a method (11) according to any one of the preceding claims.
